# EUROPÄISCHE PATENTSCHRIFT

(11) **EP 2 882 732 B1**
(45) Veröffentlichungstag und Bekanntmachung des Hinweises auf die Patenterteilung: **12.04.2017**
(21) Anmeldenummer: 13745091.2
(22) Anmeldetag: 02.08.2013
(51) Int. Cl.: C07D 401/04, A01N 43/40

(54) **HERBIZID WIRKSAME 6'-PHENYL-2,2'-BIPYRIDIN-3-CARBONSÄURE-DERIVATE**
HERBICIDALLY ACTIVE DERIVATIVES OF 6'-PHENYL-2,2'-BIPYRIDIN-3-CARBOXYLIC ACID
DÉRIVÉS DE L'ACIDE 6'-PHENYL-2,2'-BIPYRIDIN-3-CARBOXYLIQUE À ACTIVITÉ HERBICIDE

(30) Priorität: 07.08.2012 EP 12179518
(43) Veröffentlichungstag der Anmeldung: 17.06.2015
(73) Patentinhaber: Bayer CropScience AG, 40789 Monheim am Rhein (DE)
(72) Erfinder: HOFFMANN, Michael Gerhard, 65439 Flörsheim (DE); DÖLLER, Uwe, 63110 Rodgau (DE); BRÜNJES, Marco, 65795 Hattersheim am Main (DE); DIETRICH, Hansjörg, 65835 Liederbach am Taunus (DE); GATZWEILER, Elmar, 61231 Bad Nauheim (DE); ROSINGER, Christopher Hugh, 65719 Hofheim am Taunus (DE); SCHMUTZLER, Dirk, 65795 Hattersheim (DE)
(74) Vertreter: BIP Patents
(86) Internationale Anmeldenummer: PCT/EP2013/066334
(87) Internationale Veröffentlichungsnummer: WO 2014/023670

(56) Entgegenhaltungen:
- EP-A2- 0 222 254
- WO-A1-95/19358
- LAURENT BIJEIRE ET AL: "A Total Synthesis of Subarine, a Marine Alkaloid Related to the Pyridoacridine Family", EUROPEAN JOURNAL OF ORGANIC CHEMISTRY, Bd. 2004, Nr. 9, 1. Mai 2004 (2004-05-01), Seiten 1891-1893, XP055039082, ISSN: 1434-193X, DOI: 10.1002/ejoc.200400043

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Aus WO 95/19358 A1 sind herbizid wirksame Aryl- und Heteroarylpyrimidine, die einen Nicotinsäurerest tragen, bekannt. Die aus dieser Schrift bekannten Verbindungen zeigen jedoch häufig eine nicht ausreichende herbizide Wirksamkeit und/oder Verträglichkeit gegenüber Kulturpflanzen. EP 0 222 254 offenbart Nicotinsäurederivate und deren Verwendung als Herbizide. Die in EP0 222 254 beschriebenen Verbindungen zeigen jedoch nicht gegen alle Unkräuter eine zufriedenstellende Wirkung und/oder weisen keine ausreichende Verträglichkeit gegenüber den Kulturpflanzen auf. Aufgabe der vorliegenden Erfindung ist daher die Bereitstellung von weiteren herbizid wirksamen Verbindungen. Es wurde nun gefunden, dass bestimmte 6'-Phenyl-2,2'-bipyridin-3-carbonsäure-Derivate als Herbizide besonders gut geeignet sind.

Ein Gegenstand der vorliegenden Erfindung sind 6'-Phenyl-2,2'-bipyridin-3-carbonsäure-Derivate der Formel (I), deren N-Oxide und deren Salze 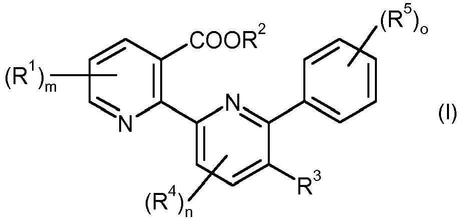 worin
R¹, R⁴ und R⁵ bedeuten unabhängig voneinander jeweils Halogen, Cyano, (C₁-C₆)-Alkyl, oder Halogen-(C₁-C₆)-alkyl,
R² bedeutet Wasserstoff oder (C₁-C₆)-Alkyl,
R³ bedeutet Wasserstoff, Halogen, (C₁-C₆)-Alkyl oder Halogen-(C₁-C₆)-alkyl,
n bedeutet 0 oder 1,
m bedeutet 0 oder 1,
o bedeutet 0, 1, 2, oder 3.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl.

Halogen steht für Fluor, Chlor, Brom oder lod.

Ist eine Gruppe mehrfach durch Reste substituiert, so ist darunter zu verstehen, daß diese Gruppe durch ein oder mehrere gleiche oder verschiedene der genannten Reste substituiert ist. Analoges gilt für den Aufbau von Ringsystemen durch verschiedene Atome und Elemente. Dabei sollen solche Verbindungen vom Anspruchsbegehren ausgenommen sein, von denen der Fachmann weiß, dass sie unter Normalbedingungen chemisch instabil sind.

Die Verbindungen der allgemeinen Formel (I) können je nach Art und Verknüpfung der Substituenten als Stereoisomere vorliegen. Sind beispielsweise ein oder mehrere asymmetrische Kohlenstoffatome vorhanden, so können Enantiomere und Diastereomere auftreten. Ebenso treten Stereoisomere auf, wenn n für 1 steht (Sulfoxide). Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfasst, jedoch nicht spezifisch definiert sind. Die erfindungsgemäßen Verbindungen können auf Grund der Oximether-Struktur auch als geometrische Isomere (E-/Z-Isomere) auftreten. Die Erfindung betrifft auch alle E-/Z-Isomere und deren Gemische, die von der allgemeinen Formel (I) umfasst, jedoch nicht spezifisch definiert sind.

Die Verbindungen der Formel (I) können Salze bilden, insbesondere für den Fall, dass für den Fall, dass R² Wasserstoff bedeutet. Salzbildung kann durch Einwirkung einer Base auf solche Verbindungen der Formel (I) erfolgen, die ein acides Wasserstoffatom tragen, z.B. im Falle von R'. Geeignete Basen sind beispielsweise organische Amine , wie Trialkylamine, Morpholin, Piperidin oder Pyridin sowie Ammonium-, Alkali- oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze.
Die Verbindungen der Formel (I) können auch durch Anlagerung einer geeigneten anorganischen oder organischen Säure, wie beispielsweise Mineralsäuren, wie beispielsweise HCl, HBr, H₂SOL₄, H₃PO₄ oder HNO₃, oder organische Säuren, z. B. Carbonsäuren, wie Ameisensäure, Essigsäure, Propionsäure, Oxalsäure, Milchsäure oder Salicylsäure oder Sulfonsäuren, wie zum Beispiel p-Toluolsulfonsäure, an eine basische Gruppe, wie z.B. Amino, Alkylamino, Dialkylamino, Piperidino, Morpholino oder Pyridino, Salze bilden. Diese Salze enthalten dann die konjugierte Base der Säure als Anion.

In allen nachfolgend genannten Formeln haben die Substituenten und Symbole, sofern nicht anders definiert, dieselbe Bedeutung wie unter Formel (I) beschrieben.

Erfindungsgemäße Verbindung können beispielsweise nach der in nachfolgendem Schema angegebenen Methode hergestellt werden.

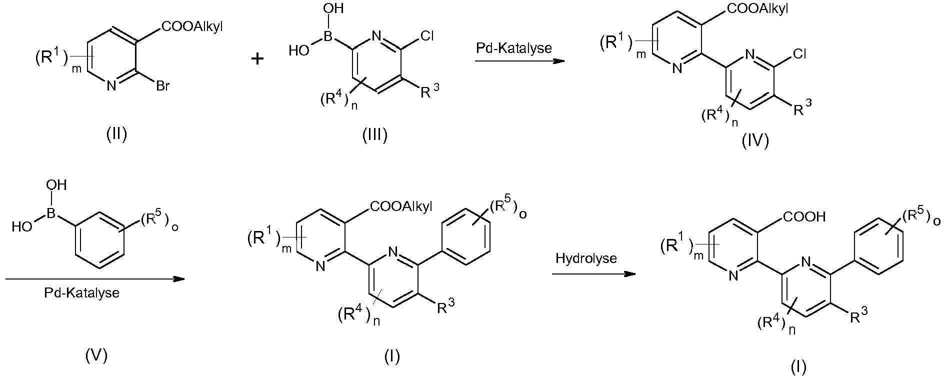

Die darin verwendeten Ausgangsmaterialien sind entweder käuflich oder nach einfachen dem Fachmann bekannten Methoden herstellbar, beispielsweise gemäß Journal of Org. Chemistry, 75(22), 7691; 2010 und Org. Synth. Coll., 1963, 4, 68.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, lowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasenunterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen in wirtschaftlich bedeutenden transgenen Kulturen von Nutz- und Zierpflanzen, z. B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Bevorzugt ist die Anwendung der erfindungsgemäßen Verbindungen oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualität auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt, siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY, oder Winnacker "Gene und Klone", VCH Weinheim 2. Auflage 1996.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227, Wolter et al., Proc. Natl. Acad. Sci. USA 85 (1988), 846-850, Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen als Herbizide zur Bekämpfung von Schadpflanzen in transgenen Kulturpflanzen.

Weiterer Gegenstand der Erfindung ist aufgrund der herbiziden Eigenschaft der Verbindungen der allgemeinen Formel (I) auch die Verwendung der erfindungsgemäßen Verbindungen der allgemeinen Formel (I) als Herbizide zur Bekämpfung von Schadpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.
Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.
Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxidaddukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix. Geeignete Safener sind beispielsweise Mefenpyr-diethyl, Cyprosulfamid, Isoxadifen-ethyl, Cloquintocet-mexyl und Dichlormid.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.
Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 15th edition, The British Crop Protection Council and the Royal Soc. of Chemistry, 2009 und dort zitierter Literatur beschrieben sind.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### A. Chemische Beispiele

### 1. Methyl-6'-chlor-2,2'-bipyridin-3-carboxylat (1)

Zu einer Lösung von 3.0 g (13.9 mmol) Methyl-2-bromnicotinat in 10 ml Dioxan gibt man unter Argon 0.49 g (0.70 mmol) (Ph₃P)₂PdCl₂ und rührt das Gemisch 30 Min. bei Raumtemperatur. Danach versetzt man die Mischung nacheinander mit 3.33 g (13.78 mmol) 2-Chlor-6-(4,4,5,5-tetramethyl-1,3,2-dioxaborolan-2-yl)pyridin, 5.76 g (41.68 mmol) K₂CO₃ und 11 ml H₂O, rührt sie 6 Stunden unter Rückfluss und lässt sie anschließend bei Raumtemperatur (RT) über Nacht stehen. Zur Aufarbeitung gibt man die Reaktionsmischung auf 150 ml H₂O und extrahiert mehrmals mit CH₂Cl₂. Die vereinigten organischen Phasen werden über Na₂SO₄ getrocknet und anschließend eingeengt. Das so erhaltene Rohprodukt wird mittels Chromatographie an Kieselgel mit Heptan/Essigsäureethylester (7:3) als Laufmittel gereinigt. Man erhält 2.50 g (72%) Produkt als farbloses Öl; ¹H-NMR (CDCl₃) δ 8.70 (dd, 1 H), 8.15 (dd, 1 H), 7.95 (dd, 1 H), 7.80 (dd, 1 H), 7.40 (dd, 1 H), 7.37 (dd, 1 H), 3.90 (s, 3H, COOMe).

### 2. Methyl-6'-(4-chlorphenyl)-2,2'-bipyridin-3-carboxylat (2)

Zu einer Lösung von 2.5 g (10.05 mmol) Methyl-6'-chlor-2,2'-bipyridin-3-carboxylat (1) in 70 ml Dioxan gibt man unter Argon 0.21 g (0.30 mmol) (Ph₃P)₂PdCl₂ und rührt das Gemisch 30 Min. bei Raumtemperatur. Danach versetzt man die Mischung nacheinander mit 1.98 g (12.08 mmol) para-Chlorphenyl-boronsäure, 4.17 g (30.17 mmol) K₂CO₃ und 7 ml H₂O, rührt sie 6 Stunden unter Rückfluss und lässt sie anschließend bei RT über Nacht stehen. Zur Aufarbeitung gibt man die Reaktionsmischung auf 100 ml H₂O und extrahiert mehrmals mit CH₂Cl₂. Die vereinigten organischen Phasen werden über Na₂SO₄ getrocknet und eingeengt. Der Rückstand wird säulenchromatographisch an Kieselgel mit Heptan/Essigsäureethylester (7:3) als Laufmittel gereinigt. Man erhält 2.7 g (82.7%) Produkt als Feststoff. Schmp.: 120.5°C; ¹H-NMR (CDCl₃) δ 8.75 (dd, 1H), 8.17 (dd, 1H), 8.00 (m, 2H, C₆H₄Cl), 7.95 (dd, 1 H), 7.92 (dd, 1 H), 7.28 (dd, 1 H), 7.45 (m, 2H, C₆H₄Cl), 7.40 (dd, 1 H), 3.60 (s, 3H, COOMe).

### 1. 6'-(4-Chlorphenyl)-2,2'-bipyridin-3-carbonsäure (3)

Eine Lösung aus 0.23 g (0.71 mmol) Methyl-6'-(4-chlorphenyl)-2,2'-bipyridin-3-carboxylat (2) und 0.04 g (0.99 mmol) NaOH in 6 ml THF und 2.5 ml H₂O wird 4 Stunden bei 50°C gerührt und anschließend 12 Stunden bei RT stehen gelassen. Zur Aufarbeitung wird THF abrotiert und der wässrige Rückstand mit wenig CH₂Cl₂ extrahiert. Die wässrige Phase wird mit 1 N HCl auf pH 2 gestellt, wobei ein zäher Kristallbrei als Produkt anfällt. Man erhält 70 mg (32%) Produkt als Feststoff; Schmp.: 221°C; ¹H-NMR (CDCl₃) δ 8.85 (dd, 1 H), 8.79 (dd, 1 H), 8.62 (dd, 1H), 8.15 (dd, 1 H), 7.85 (m, 2H, C₆H₄Cl), 7.82 (dd, 1 H), 7.54 (m, 2H, C₆H₄Cl), 7.51 (d, 1 H).

### 2. Kalium-6'-(4-chlorphenyl)-2,2'-bipyridin-3-carbonsäure (4)

Zu einer Lösung von 0.042 g (0.13 mmol) 6'-(4-Chlorphenyl)-2,2'-bipyridin-3-carbonsäure (3) in 8 ml eines Lösemittelgemischs aus Dioxan/Methanol (8/2) gibt man 0.008 g KOH in 10 ml MeOH/H₂O (1/1) gelöst zu und rührt dieses Gemisch 1 Stunde bei 40°C. Danach wird die Lösung bis zur Trockene eingeengt. Man erhält 40 mg (93%) Produkt als amorphen Feststoff. ¹H-NMR (DMSO) δ 8.41 (dd, 1 H), 8.30 (m, 2H, C₆H₄Cl), 7.90 (m, 2H), 7.75 (d, 2H), 7.70 (d, 2H), 7.50 (m, 2H, C₆H₄Cl), 7.25 (dd, 1 H).

Die in der nachfolgenden Tabelle aufgeführten Beispiele wurden analog oben genannten Methoden hergestellt beziehungsweise sind analog oben genannten Methoden erhältlich. Die darin aufgeführten Verbindungen sind ganz besonders bevorzugt. Die Abkürzung Me bedeutet Methyl. Sind für den Rest R² die Symbole K oder Na angegeben, so bedeutet dies, dass die jeweilige erfindungsgemäße Verbindung in Form ihres Natrium- oder Kaliumsalzes vorliegt.

**Tabelle 1: Erfindungsgemäße Verbindungen der Formel (I), worin R¹, R³ und R⁴ jeweils für Wasserstoff stehen, und R² und R⁵ die in Tabelle 1 angegebenen Bedeutungen haben**

| | | |
|---|---|---|
| 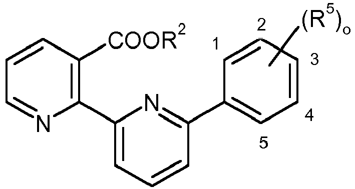 | | |

| Bsp.-Nr. | R² | R⁵ |
|---|---|---|
| 1.001 | Me | -- |
| 1.002 | Me | 3-Cl |
| 1.003 | Me | 3,4-Cl₂ |
| 1.004 | Me | 3,4,5-Cl₃ |
| 1.005 | Me | 3,5-Cl₂ |
| *1.006 | Me | 3,5-Cl₂-4-OMe |
| 1.007 | Me | 3,5-Cl₂-4-F |
| 1.008 | Me | 2,4-Cl₂ |
| 1.009 | Me | 2,3,4-Cl₃ |
| 1.010 | Me | 2,4-Cl₂-3-F |
| *1.011 | Me | 2,4-Cl₂-3-OMe |
| 1.012 | Me | 2,4-Cl₂-3-Me |
| 1.013 | Me | 2,4,5-Cl₃ |
| 1.014 | Me | 2-Cl-4-F |
| *1.015 | Me | 2-Cl-4-OMe |
| 1.016 | Me | 2-Cl-4-Me |
| 1.017 | Me | 3-F |
| 1.018 | Me | 4-CI-3-F |
| 1.019 | Me | 3,4-F₂ |
| 1.020 | Me | 3,4,5-F₃ |
| *1.021 | Me | 3,5-F₂-4-Me |
| *1.022 | Me | 3,5-F₂-4-OMe |
| 1.023 | Me | 2,4-F₂ |
| 1.024 | Me | 3-Cl-4-F |
| *1.025 | Me | 2,4-F₂-3-OMe |
| *1.026 | Me | 2-F-4-OMe |
| 1.027 | Me | 4-Cl-2-F |
| 1.028 | Me | 2,3,5-F₃ |
| 1.029 | Me | 5-Cl-2,3-F₂ |
| 1.030 | Me | 2-F-4-Me |
| *1.031 | Me | 5-Cl-3-F-4-OMe |
| *1.032 | Me | 3,5-F₂-4-NMe₂ |
| *1.033 | Me | 5-Cl-3-F-4-NMe₂ |
| *1.034 | Me | 2-F-4-NMe₂ |
| *1.035 | Me | 2-F-4-NH₂ |
| 1.036 | Me | 3,4-Cl₂-2-F |
| 1.037 | Me | 3,4,5-Cl₃-2-F |
| *1.038 | Me | 2,5-F₂-4-OMe |
| *1.039 | Me | 5-Cl-2-F-4-OMe |
| 1.040 | Me | 3-CF₃ |
| 1.041 | Me | 2-Cl-3-CF₃ |
| 1.042 | Me | 2,4-Cl₂-3-CF₃ |
| 1.043 | Me | 4-Cl-3-CF₃ |
| 1.044 | Me | 5-Cl-3-CF₃ |
| 1.045 | Me | 3-CF₃-2-F |
| 1.046 | Me | 3-CF₃-2,4-F₂ |
| 1.047 | Me | 3-CF₃-4-F |
| 1.048 | Me | 3-CF₃-5-F |
| 1.049 | Me | 4-CF₃ |
| 1.050 | Me | 3-Cl-4-CF₃ |
| 1.051 | Me | 3,5-Cl₂-4CF₃ |
| 1.052 | Me | 5-Cl-4-CF₃ |
| *1.053 | Me | 4-SMe |
| *1.054 | Me | 3-Cl-4-SMe |
| *1.055 | Me | 5-Cl-4-SMe |
| *1.056 | Me | 3,5-Cl₂-4-SMe |
| *1.057 | Me | 3-F-4-SMe |
| *1.058 | Me | 3,5-F₂-4-SMe |
| *1.059 | Me | 5-F-4-SMe |
| *1.060 | Me | 3-Cl-5-F-4-SMe |
| 1.061 | H | - |
| 1.062 | H | 3-Cl |
| 1.063 | H | 3,4-Cl₂ |
| 1.064 | H | 3,4,5-Cl₃ |
| 1.065 | H | 3,5-Cl₂ |
| *1.066 | H | 3,5-Cl₂-4-OMe |
| 1.067 | H | 3,5-Cl₂-4-F |
| 1.068 | H | 2,4-Cl₂ |
| 1.069 | H | 2,3,4-Cl₃ |
| 1.070 | H | 2,4-Cl₂-3-F |
| *1.071 | H | 2,4-Cl₂-3-OMe |
| 1.072 | H | 2,4-Cl₂-3-Me |
| 1.073 | H | 2,4,5-Cl₃ |
| 1.074 | H | 2-Cl-4-F |
| *1.075 | H | 2-Cl-4-OMe |
| 1.076 | H | 2-Cl-4-Me |
| 1.077 | H | 3-F |
| 1.078 | H | 4-Cl-3-F |
| 1.079 | H | 3,4-F₂ |
| 1.080 | H | 3,4,5-F₃ |
| 1.081 | H | 3,5-F₂-4-Me |
| *1.082 | H | 3,5-F₂-4-OMe |
| 1.083 | H | 2,4-F₂ |
| 1.084 | H | 3-Cl-4-F |
| *1.085 | H | 2,4-F₂-3-OMe |
| *1.086 | H | 2-F-4-OMe |
| 1.087 | H | 4-Cl-2-F |
| 1.088 | H | 2,3,5-F₃ |
| 1.089 | H | 5-Cl-2,3-F₂ |
| 1.090 | H | 2-F-4-Me |
| *1.091 | H | 5-Cl-3-F-4-OMe |
| *1.092 | H | 3,5-F₂-4-NMe₂ |
| *1.093 | H | 5-Cl-3-F-4-NMe₂ |
| *1.094 | H | 2-F-4-NMe₂ |
| *1.095 | H | 2-F-4-NH₂ |
| 1.096 | H | 3,4-Cl₂-2-F |
| 1.097 | H | 3,4,5-Cl₃-2-F |
| *1.098 | H | 2,5-F₂-4-OMe |
| *1.099 | H | 5-Cl-2-F-4-OMe |
| 1.100 | H | 3-CF₃ |
| 1.101 | H | 2-Cl-3-CF₃ |
| 1.102 | H | 2,4-Cl₂-3-CF₃ |
| 1.103 | H | 4-Cl-3-CF₃ |
| 1.104 | H | 5-Cl-3-CF₃ |
| 1.105 | H | 3-CF₃-2-F |
| 1.106 | H | 3-CF₃-2,4-F₂ |
| 1.107 | H | 3-CF₃-4-F |
| 1.108 | H | 3-CF₃-5-F |
| 1.109 | H | 4-CF₃ |
| 1.110 | H | 3-Cl-4-CF₃ |
| 1.111 | H | 3,5-Cl₂-4CF₃ |
| 1.112 | H | 5-Cl-4-CF₃ |
| *1.113 | H | 4-SMe |
| *1.114 | H | 3-Cl-4-SMe |
| *1.115 | H | 5-Cl-4-SMe |
| *1.116 | H | 3,5-Cl₂-4-SMe |
| *1.117 | H | 3-F-4-SMe |
| *1.118 | H | 3,5-F₂-4-SMe |
| *1.119 | H | 5-F-4-SMe |
| 1.120 | H | 3-Cl-5-F-4-SMe |
| 1.121 | Na | -- |
| 1.122 | Na | 3-Cl |
| 1.123 | Na | 3,4-Cl₂ |
| 1.124 | Na | 3,4,5-Cl₃ |
| 1.125 | Na | 3,5-Cl₂ |
| *1.126 | Na | 3,5-Cl₂-4-OMe |
| 1.127 | Na | 3,5-Cl₂-4-F |
| 1.128 | Na | 2,4-Cl₂ |
| 1.129 | Na | 2,3,4-Cl₃ |
| 1.130 | Na | 2,4-Cl₂-3-F |
| *1.131 | Na | 2,4-Cl₂-3-OMe |
| 1.132 | Na | 2,4-Cl₂-3-Me |
| 1.133 | Na | 2,4,5-Cl₃ |
| 1.134 | Na | 2-Cl-4-F |
| *1.135 | Na | 2-Cl-4-OMe |
| 1.136 | Na | 2-Cl-4-Me |
| 1.137 | Na | 3-F |
| 1.138 | Na | 4-Cl-3-F |
| 1.139 | Na | 3,4-F₂ |
| 1.140 | Na | 3,4,5-F₃ |
| 1.141 | Na | 3,5-F₂-4-Me |
| *1.142 | Na | 3,5-F₂-4-OMe |
| 1.143 | Na | 2,4-F₂ |
| 1.144 | Na | 3-Cl-4-F |
| *1.145 | Na | 2,4-F₂-3-OMe |
| *1.146 | Na | 2-F-4-OMe |
| 1.147 | Na | 4-Cl-2-F |
| 1.148 | Na | 2,3,5-F₃ |
| 1.149 | Na | 5-Cl-2,3-F₂ |
| 1.150 | Na | 2-F-4-Me |
| *1.151 | Na | 5-Cl-3-F-4-OMe |
| *1.152 | Na | 3,5-F₂-4-NMe₂ |
| *1.153 | Na | 5-Cl-3-F-4-NMe₂ |
| *1.154 | Na | 2-F-4-NMe₂ |
| *1.155 | Na | 2-F-4-NH₂ |
| 1.156 | Na | 3,4-Cl₂-2-F |
| 1.157 | Na | 3,4,5-Cl₃-2-F |
| *1.158 | Na | 2,5-F₂-4-OMe |
| *1.159 | Na | 5-Cl-2-F-4-OMe |
| 1.160 | Na | 3-CF₃ |
| 1.161 | Na | 2-Cl-3-CF₃ |
| 1.162 | Na | 2,4-Cl₂-3-CF₃ |
| 1.163 | Na | 4-Cl-3-CF₃ |
| 1.164 | Na | 5-Cl-3-CF₃ |
| 1.165 | Na | 3-CF₃-2-F |
| 1.166 | Na | 3-CF₃-2,4-F₂ |
| 1.167 | Na | 3-CF₃-4-F |
| 1.168 | Na | 3-CF₃-5-F |
| 1.169 | Na | 4-CF₃ |
| 1.170 | Na | 3-Cl-4-CF₃ |
| 1.171 | Na | 3,5-Cl₂-4CF₃ |
| 1.172 | Na | 5-Cl-4-CF₃ |
| *1.173 | Na | 4-SMe |
| *1.174 | Na | 3-Cl-4-SMe |
| *1.175 | Na | 5-Cl-4-SMe |
| *1.176 | Na | 3,5-Cl₂-4-SMe |
| *1.177 | Na | 3-F-4-SMe |
| *1.178 | Na | 3,5-F₂-4-SMe |
| *1.179 | Na | 5-F-4-SMe |
| 1.180 | Na | 3-Cl-5-F-4-SMe |
| 1.181 | K | -- |
| 1.182 | K | 3-Cl |
| 1.183 | K | 3,4-Cl₂ |
| 1.184 | K | 3,4,5-Cl₃ |
| 1.185 | K | 3,5-Cl₂ |
| *1.186 | K | 3,5-Cl₂-4-OMe |
| 1.187 | K | 3,5-Cl₂-4-F |
| 1.188 | K | 2,4-Cl₂ |
| 1.189 | K | 2,3,4-Cl₃ |
| 1.190 | K | 2,4-Cl₂-3-F |
| *1.191 | K | 2,4-Cl₂-3-OMe |
| 1.192 | K | 2,4-Cl₂-3-Me |
| 1.193 | K | 2,4,5-Cl₃ |
| 1.194 | K | 2-Cl-4-F |
| *1.195 | K | 2-Cl-4-OMe |
| 1.196 | K | 2-Cl-4-Me |
| 1.197 | K | 3-F |
| 1.198 | K | 4-Cl-3-F |
| 1.199 | K | 3,4-F₂ |
| 1.200 | K | 3,4,5-F₃ |
| 1.201 | K | 3,5-F₂-4-Me |
| *1.202 | K | 3,5-F₂-4-OMe |
| 1.203 | K | 2,4-F₂ |
| 1.204 | K | 3-Cl-4-F |
| *1.205 | K | 2,4-F₂-3-OMe |
| *1.206 | K | 2-F-4-OMe |
| 1.207 | K | 4-Cl-2-F |
| 1.208 | K | 2,3,5-F₃ |
| 1.209 | K | 5-Cl-2,3-F₂ |
| 1.210 | K | 2-F-4-Me |
| *1.211 | K | 5-Cl-3-F-4-OMe |
| *1.212 | K | 3,5-F₂-4-NMe₂ |
| *1.213 | K | 5-Cl-3-F-4-NMe₂ |
| *1.214 | K | 2-F-4-NMe₂ |
| *1.215 | K | 2-F-4-NH₂ |
| 1.216 | K | 3,4-Cl₂-2-F |
| *1.217 | K | 3,4,5-Cl₃-2-F |
| *1.218 | K | 2,5-F₂-4-OMe |
| 1.219 | K | 5-Cl-2-F-4-OMe |
| 1.220 | K | 3-CF₃ |
| 1.221 | K | 2-Cl-3-CF₃ |
| 1.222 | K | 2,4-Cl₂-3-CF₃ |
| 1.223 | K | 4-Cl-3-CF₃ |
| 1.224 | K | 5-Cl-3-CF₃ |
| 1.225 | K | 3-CF₃-2-F |
| 1.226 | K | 3-CF₃-2,4-F₂ |
| 1.227 | K | 3-CF₃-4-F |
| 1.228 | K | 3-CF₃-5-F |
| 1.229 | K | 4-CF₃ |
| 1.230 | K | 3-Cl-4-CF₃ |
| 1.231 | K | 3,5-Cl₂-4CF₃ |
| 1.232 | K | 5-Cl-4-CF₃ |
| *1.233 | K | 4-SMe |
| *1.234 | K | 3-Cl-4-SMe |
| *1.235 | K | 5-Cl-4-SMe |
| *1.236 | K | 3,5-Cl₂-4-SMe |
| *1.237 | K | 3-F-4-SMe |
| *1.238 | K | 3,5-F₂-4-SMe |
| *1.239 | K | 5-F-4-SMe |
| *1.240 | K | 3-Cl-5-F-4-SMe |
| 1.241 | Me | 3-Cl-5-F |
| 1.242 | K | 3-Cl-5-F |

| | | |
|---|---|---|
| * = nicht Gegenstand der Erfindung | | |

### B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I) und/oder deren Salze,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I) und/oder deren Salze,
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wäßrige Suspension bzw.

Emulsion mit einer Wasseraufwandmenge von umgerechnet 600 bis 800 l/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen). Dabei zeigen beispielsweise die Verbindungen Nr. 1.002, 1.182 und 1.242 bei einer Aufwandmenge von 1280 g/ha jeweils eine mindestens 90%-ige Wirkung gegen Viola tricolor und Amaranthus retroflexus auf.

### 2. Herbizide Wirkung gegen Schadpflanzen im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen). Dabei zeigen beispielsweise die Verbindungen Nr. 1.002, 1.241, 1.182 und 1.242 bei einer Aufwandmenge von 320 g/ha jeweils eine mindestens 80%-ige Wirkung gegen Amaranthus retroflexus.

## Patentansprüche

1. 6'-Phenyl-2,2'-bipyridin-3-carbonsäure-Derivate der Formel (I), deren N-Oxide und deren Salze 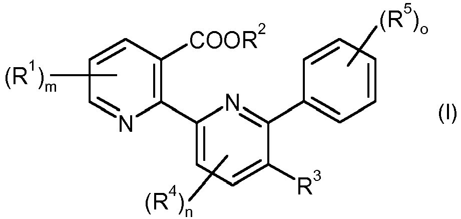 worin
R¹, R⁴ und R⁵ bedeuten unabhängig voneinander jeweils Halogen, Cyano, (C₁-C₄)-Alkyl, oder Halogen-(C₁-C₄)-alkyl,
R² bedeutet Wasserstoff oder (C₁-C₆)-Alkyl,
R³ bedeutet Wasserstoff, Halogen, (C₁-C₆)-Alkyl oder Halogen-(C₁-C₆)-alkyl,
n bedeutet 0 oder 1,
m bedeutet 0 oder 1,
o bedeutet 0, 1, 2 oder 3.

2. Herbizide Mittel, **gekennzeichnet durch** einen herbizid wirksamen Gehalt an mindestens einer Verbindung der Formel (I) gemäß Anspruch 1.

3. Herbizide Mittel nach Anspruch 2 in Mischung mit Formulierungshilfsmitteln.

4. Herbizide Mittel nach Anspruch 2 oder 3 enthaltend mindestens einen weiteren pestizid wirksamen Stoff aus der Gruppe Insektizide, Akarizide, Herbizide, Fungizide, Safener und Wachstumsregulatoren.

5. Herbizide Mittel nach Anspruch 4 enthaltend einen Safener.

6. Herbizide Mittel nach Anspruch 5 enthaltend Cyprosulfamid, Cloquintocet-mexyl, Mefenpyr-diethyl oder Isoxadifen-ethyl.

## Claims

1. 6'-Phenyl-2,2'-bipyridine-3-carboxylic acid derivatives of the formula (I), their N-oxides and their salts 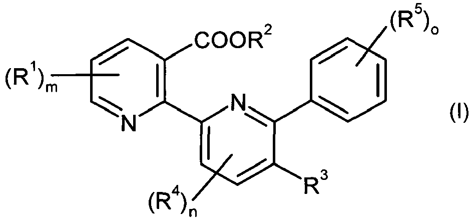 in which
R¹, R⁴ and R⁵ independently of one another are in each case halogen, cyano, (C₁-C₄)-alkyl, or halo-(C₁-C₄)-alkyl,
R² is hydrogen or (C₁-C₆)-alkyl,
R³ is hydrogen, halogen, (C₁-C₆)-alkyl or halo-(C₁-C₆)-alkyl,
n is 0 or 1,
m is 0 or 1,
o is 0, 1, 2 or 3.

2. Herbicidal compositions **characterized by** a herbicidally active content of at least one compound of the formula (I) according to Claim 1.

3. Herbicidal compositions according to Claim 2 in a mixture with formulation auxiliaries.

4. Herbicidal compositions according to Claim 2 or 3, comprising at least one further pesticidally active substance from the group of insecticides, acaricides, herbicides, fungicides, safeners and growth regulators.

5. Herbicidal compositions according to Claim 4, comprising a safener.

6. Herbicidal compositions according to Claim 5, comprising cyprosulfamide, cloquintocet-mexyl, mefenpyr-diethyl or isoxadifen-ethyl.

## Revendications

1. Dérivés de l'acide 6'-phényl-2,2'-bipyridine-3-carboxylique de formule (I), leurs N-oxydes et leurs sels 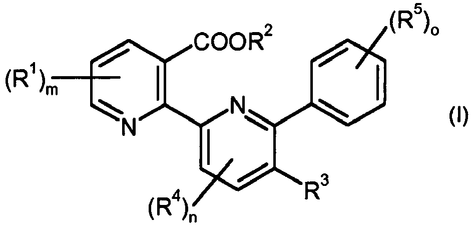 dans laquelle
R¹, R⁴ et R⁵ signifient, indépendamment les uns des autres, à chaque fois halogène, cyano, (C₁-C₄)-alkyle, ou halogéno-(C₁-C₄)-alkyle,
R² signifie hydrogène ou (C₁-C₆)-alkyle,
R³ signifie hydrogène, halogène, (C₁-C₆)-alkyle ou halogéno-(C₁-C₆)-alkyle,
n signifie 0 ou 1,
m signifie 0 ou 1,
o signifie 0, 1, 2 ou 3.

2. Agents herbicides, **caractérisés par** une teneur active en tant qu'herbicide en au moins un composé de formule (I) selon la revendication 1.

3. Agents herbicides selon la revendication 2 en mélange avec des adjuvants de formulation.

4. Agents herbicides selon la revendication 2 ou 3 contenant au moins une autre substance active en tant que pesticide du groupe formé par les insecticides, les acaricides, les herbicides, les fongicides, les antidotes et les régulateurs de croissance.

5. Agents herbicides selon la revendication 4 contenant un antidote.

6. Agents herbicides selon la revendication 5, contenant du cyprosulfamide, du cloquintocet-mexyl, du méfenpyr-diéthyl ou de l'isoxadifen-éthyl.
